(19) Europäisches Patentamt European Patent Office Office européen des brevets

(11) **EP 3 984 041 B1**

(12) **EUROPEAN PATENT SPECIFICATION**

(45) Date of publication and mention of the grant of the patent:
**24.01.2024 Bulletin 2024/04**

(21) Application number: **20736921.6**

(22) Date of filing: **17.06.2020**

(51) International Patent Classification (IPC):
*G16H 10/40* (2018.01)   *G16H 50/50* (2018.01)
*G01N 33/48* (2006.01)   *G01N 13/04* (2006.01)
*G01N 33/49* (2006.01)

(52) Cooperative Patent Classification (CPC):
**G16H 10/40; G16H 50/50**

(86) International application number:
**PCT/EP2020/066804**

(87) International publication number:
**WO 2020/254431 (24.12.2020 Gazette 2020/52)**

(54) **METHOD AND SYSTEM FOR DATING BLOOD POOLS**

VERFAHREN UND SYSTEM ZUR DATIERUNG VON BLUTFLECKEN

PROCÉDÉ ET SYSTÈME DE DATATION DE DÉPÔTS DE SANG

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.06.2019 EP 19305772**

(43) Date of publication of application:
**20.04.2022 Bulletin 2022/16**

(73) Proprietors:
• **Universite d'Aix Marseille**
  **13284 Marseille Cedex 07 (FR)**
• **Centre National de la Recherche Scientifique**
  **75016 Paris (FR)**
• **Etat Francais Represente par le Ministere de l'Interieur**
  **92130 Issy-les Moulineaux (FR)**

(72) Inventors:
• **BRUTIN, David**
  **13009 Marseille (FR)**
• **NICLOUX, Céline**
  **95300 PONTOISE (FR)**
• **SMITH, Fiona**
  **13004 MARSEILLE (FR)**

(74) Representative: **Osha BWB**
  **2, rue de la Paix**
  **75002 Paris (FR)**

(56) References cited:
• **THANAKIATKRAI P ET AL: "Age estimation of bloodstains using smartphones and digital image analysis", FORENSIC SCIENCE INTERNATIONAL, ELSEVIER B.V, AMSTERDAM, NL, vol. 233, no. 1, 9 October 2013 (2013-10-09), pages 288-297, XP028794918, ISSN: 0379-0738, DOI: 10.1016/J.FORSCIINT.2013.09.027 cited in the application**
• **CHOI W ET AL: "Highly sensitive and accurate estimation of bloodstain age using smartphone", BIOSENSORS AND BIOELECTRONICS, vol. 130, 1 April 2019 (2019-04-01), pages 414-419, XP055644236, AMSTERDAM, NL ISSN: 0956-5663, DOI: 10.1016/j.bios.2018.09.017**
• **DOTY K C ET AL: "A Raman "spectroscopic clock" for bloodstain age determination: the first week after deposition", ANALYTICAL AND BIOANALYTICAL CHEMISTRY, SPRINGER, DE, vol. 408, no. 15, 23 March 2016 (2016-03-23), pages 3993-4001, XP035867780, ISSN: 1618-2642, DOI: 10.1007/S00216-016-9486-Z [retrieved on 2016-03-23]**

- EDELMAN G J ET AL: "Practical Implementation of Blood Stain Age Estimation Using Spectroscopy", IEEE JOURNAL OF SELECTED TOPICS IN QUANTUM ELECTRONICS, IEEE SERVICE CENTER, PISCATAWAY, NJ, US, vol. 22, no. 3, 1 May 2016 (2016-05-01), pages 1-7, XP011611900, ISSN: 1077-260X, DOI: 10.1109/JSTQE.2016.2536655 [retrieved on 2016-05-25]
- LAAN N ET AL: "Morphology of drying blood pools", FORENSIC SCIENCE INTERNATIONAL, ELSEVIER B.V, AMSTERDAM, NL, vol. 267, 24 August 2016 (2016-08-24), pages 104-109, XP029761579, ISSN: 0379-0738, DOI: 10.1016/J.FORSCIINT.2016.08.005 cited in the application
- BACCHIN P ET AL: "Drying colloidal systems: Laboratory models for a wide range of applications", EUROPEAN PHYSICAL JOURNAL E. SOFT MATTER, EDP SCIENCES, IT, vol. 41, no. 8, 22 August 2018 (2018-08-22), pages 1-34, XP036577123, ISSN: 1292-8941, DOI: 10.1140/EPJE/I2018-11712-X [retrieved on 2018-08-22]

**Description**

Field of the disclosure

The present disclosure concerns, but is not limited to, a computer-implemented method and a system for dating blood pools lying on a substrate.

Technical background

Despite the recent development of forensic science, estimating bloodstain age has not yet been convincingly implemented in routine crime scene investigation. Current methods for estimating blood pools age rely on complex procedures requiring specialist knowledge and/or sophisticated, expensive machines. Spectroscopy-based techniques and hyperspectral imaging are currently the techniques closest to implementation due to their widespread availability. However, both types of techniques suffer from low accuracy, are destructive and non-easily transportable on every crime scene.

The article by Laan et al., Morphology of drying blood pools, Forensic Sci. Int. 267, pages 104-109, 2016, attempts to characterize the drying process of blood pools and identifies different drying stages. However, this attempt represents only a very preliminary step towards the establishment of a predictive drying model ideally aimed at leading to an accurate determination of the time elapsed between a given time at which a blood pool is considered and a time at which the blood pool has been spread. Indeed, the various characteristics of a blood pool, such as for example the size and shape thereof, together with the variability of the environmental conditions, lead overall to a daunting challenge in establishing such drying model, since no apparent correlation exists that could weight all cited blood pool characteristics and environmental conditions.

The article by Choi et al., Highly sensitive and accurate estimation of bloodstain age using smartphone, Biosens. Bioelectron. 130, pages 414-419, 2019, describes a system for estimating the age of bloodstains that combines three analyses, namely blood pool analysis, crack ratio analysis and colorimetric analysis, which are all based on the image analysis of a bloodstain. Parameters are extracted from such image analysis and the extracted parameters are then compared to corresponding parameters of reference images stored in a database.

Another method for estimating the age of bloodstains is described in the article by Thanakiatkrai et al., Age estimation of bloodstains using smartphones and digital image analysis, Forensic Sci. Int. 233, pages 288-297, 2013. Such method is based on colorimetry and plots color parameters extracted from digital images of bloodstains against time, and then compares the results to a calibration curve. The effects of further parameters on the estimation, such as the type of smartphone camera, person-to-person variation, temperature, humidity, light exposure, anticoagulant, and substrate, are further discussed in the article.

All prior art methods merely provide rough age estimations, and lack a drying model reliably representing a blood pool's drying process. There thus still exists a need for a method offering a high accuracy and reliability in dating blood pools, while remaining simple to be implemented, not destructive or altering, and transferable on the field.

Summary of the disclosure

In what follows, the term "comprise" is synonym of (means the same as) "include" and "contains", is inclusive and open and does not exclude other non-recited elements. Moreover, in the present disclosure, the terms "about" and "substantially" are synonyms of (mean the same as) a margin less and/or more than to 10% of the respective value.

One of the objectives of the present disclosure is to provide a method for dating blood pools lying on a substrate.

According to the invention, such an objective is attained by a computer implemented method for dating a blood pool lying on a substrate, comprising:

- determining, based on at least one picture of a blood pool lying on a substrate taken at a given time $t$, the drying front of the blood pool at the given time; and
- determining, based on the determined drying front of the blood pool at the given time $t$ and on a drying model, the time elapsed between the given time $t$ and an initial time $t_0$ at which the blood pool initiated a drying process on the substrate,

wherein determining the drying front of the blood pool at the given time $t$ comprises determining the wet perimeter $P_t$ of the blood pool at the given time $t$, and
wherein the drying model correlates the time elapsed with a blood's diffusion coefficient $D_{blood}$ measured for a plurality of predetermined environmental conditions and a plurality of initial blood pool conditions.
wherein the time elapsed between the given time $t$ and the initial time $t_0$ is determined by

- providing a database comprising sets of predetermined environmental conditions comprising at least the temperature and humidity of the environment to which a blood pool is exposed, and the nature of the substrate on which a blood pool lies, and initial blood pool conditions including initial areas $A_0$, the sets of predetermined environmental and initial blood pool conditions being associated with corresponding sets of measured blood pool parameters including blood's diffusion coefficients $D_{blood}$, blood pool's heights h, and correlation coefficients a and β,

> wherein the measured blood pool parameters of each set of measured blood pool parameters are measured at the environmental and initial blood pool conditions of the associated set of predetermined environmental and initial blood pool conditions, and
>
> wherein the correlation coefficients a and β are measured for a predetermined number of blood pools having different masses, and provided by a fitting curve deduced from the superimposition of a predetermined number of corresponding variations of a normalized mass $m_t/m_0$ as functions of a normalized wet area $A_t/A_0$, wherein $m_t$ is the mass variation of the blood pool at the given time t, and $m_0$ is the mass of the blood pool at the initial time $t_0$,

- determining the environmental conditions comprising the temperature and humidity of the environment to which the blood pool is exposed at the given time t and the nature of the substrate on which the blood pool lies, and an initial area $A_0$ of the blood pool corresponding to the area of the blood pool at the initial time $t_0$,
- comparing the determined environmental conditions and the determined initial area $A_0$ with the sets of predetermined environmental and initial blood pool conditions, and
- determining a set of measured blood pool parameters comprising the blood's diffusion coefficient $D_{blood}$, the blood pool's height h and the correlation coefficients a and β, which are associated with the set of predetermined environmental and initial blood pool conditions identical to the determined environmental conditions and the determined initial area $A_0$.

The invention also provides a system therefor, as defined in appended claim 8.

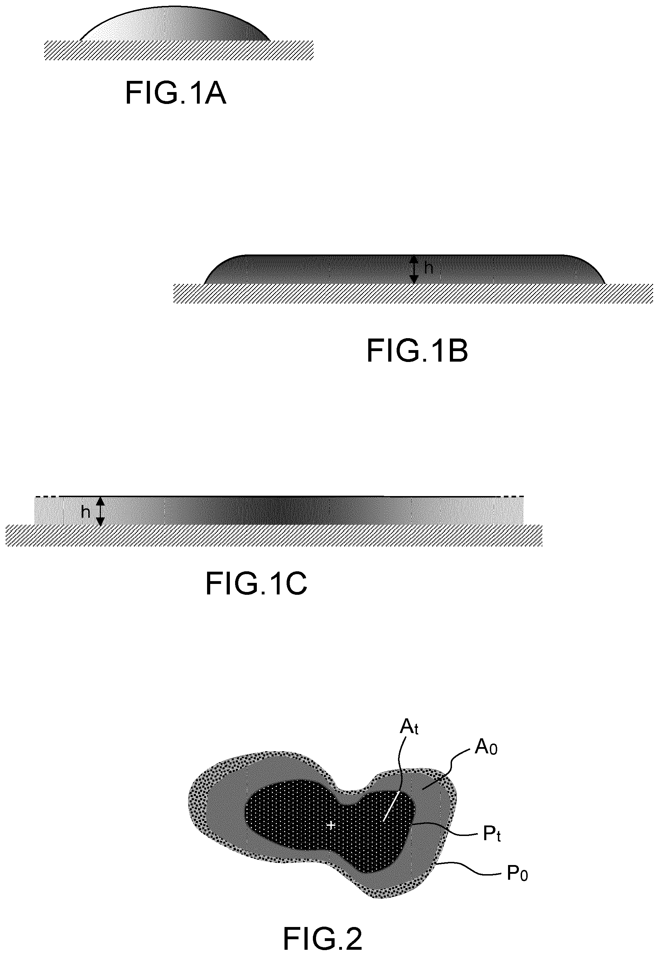

In the present description and in the claims, by "blood pool", it is meant any blood configuration (Figure 1B) intermediate between a drop (Figure 1A), which has a curved surface, and an infinite stretch of liquid having a flat surface (Figure 1C), as illustrated by Figure 1. According to one or more embodiments, a blood pool presents a substantially flat, limited surface.

The method of the disclosure may be applied to any blood pool drying on a substrate, independently from the volume thereof. However, according to one or more embodiments, the method is particularly adapted to blood pools having a volume greater than 170 μL. Indeed, when the blood pool has a volume smaller than 170 μL, for substrates giving a contact angle of about 45° and for a surface tension at the gas-liquid interface of 60 mPa·s, the blood configuration tends to take the shape of a drop.

The method according to the present disclosure can for example be successfully implemented in routine crime scene investigation, by providing a real-time, accurate estimation of the time elapsed after a victim's death, i.e. the postmortem interval (PMI), and/or the approximate time at which blood was spread on a substrate and thus at which a crime involving bleeding was committed.

Hence, by determining, based on a drying front of the blood pool, which is simply determined on the basis of one or more pictures, and based on a drying model, the method according to the disclosure provides a real-time and accurate estimation of the time at which the blood of a victim, and/or a culprit, was spilled.

By drying process, it is meant a process which comprises at least one evaporation process. The drying process initiated by the blood pool comprises the evaporation of water contained within the blood of the blood pool. Such drying process may be function of the environmental conditions to which the blood pool is exposed.

According to the invention, the drying model correlates the time elapsed with a blood's diffusion coefficient $D_{blood}$ by means of blood pool parameters including the blood pool's height h.

According to the invention, the time elapsed between the given time t and the initial time $t_0$ is determined by:

- providing a database comprising sets of predetermined environmental and initial blood pool conditions including initial areas $A_0$, the sets of predetermined environmental and initial blood pool conditions being associated with corresponding sets of measured blood pool parameters including blood's diffusion coefficients $D_{blood}$ and blood pool's heights h,
- determining the environmental conditions to which the blood pool is exposed at the given time t, and an initial area $A_0$ of the blood pool corresponding to the area of the blood pool at the initial time $t_0$,
- comparing the determined environmental conditions and the determined initial area $A_0$ with the sets of predetermined environmental and initial blood pool conditions, and
- determining a set of measured blood pool parameters comprising the blood's diffusion coefficient $D_{blood}$ and the blood pool's height h associated with the set of predetermined environmental and initial blood pool conditions matching with the determined environmental conditions and the determined initial area $A_0$.

According to the invention, the measured blood pool parameters including blood's diffusion coefficients $D_{blood}$ and blood pool's heights h included in the sets associated with the corresponding sets of predetermined environmental and initial blood pool conditions comprised in the database, are measured at the predetermined environmental and initial blood pool conditions of the corresponding associated set.

According to the invention, the predetermined environmental conditions comprise at least the temperature and humidity to which the blood pool is exposed, and the nature of the substrate on which the substrate lies.

According to the invention, determining the time elapsed between the given time and a time at which the blood pool initiated a drying process on the substrate is done by calculation of such time based on a predetermined mathematical equation representing a predetermined drying model.

According to one or more embodiments, the drying model comprises a function correlating the time elapsed between the given time t and the initial time $t_0$ with the blood's diffusion coefficient $D_{blood}$, the blood's diffusion coefficient $D_{blood}$ having a substantially constant value during the time elapsed between the given time t and the initial time $t_0$ for the same environmental conditions.

According to one or more embodiments, the blood's diffusion coefficient is expressed according to the following equation:

$$D_{blood} = K_i L_k L^{*0.5} \qquad (1)$$

wherein:

- $D_{blood}$ is the blood's diffusion coefficient;
- $K_i$ is a coefficient of transfer from the liquid state into the gaseous state and is expressed according to the following equation:

$$K_i = J^* \frac{RT}{MP_w} \qquad (2)$$

wherein:

- M is the molecular weight of water;
- $P_w$ is the saturation vapor pressure of water at the surface of the blood pool;
- R is the ideal gas constant;
- T is the temperature of the environment to which the blood pool is exposed; and
- J* is the evaporation rate of the blood pool and is expressed according to the following equation:

$$J^* = \frac{\delta m}{\delta t} \times \frac{1}{A_0} \qquad (3)$$

wherein:

- $\delta m/\delta t$ is the mass variation of the blood pool with time; and
- $A_0$ is the area of the blood pool at a time $t_0$ at which the blood pool initiated a drying process on the substrate;

- $L_k$ is the Knudsen layer and is expressed according to the following equation:

$$L_k = \frac{k_B T}{\pi d^2 P_a} \qquad (4)$$

wherein:

- $k_B$ is Boltzmann's constant;
- d is the molecular diameter of water molecules; and

- $P_a$ is the atmospheric pressure;

- L* is a shape factor and is expressed according to the following equation:

$$L^* = \frac{A_0}{P_t h} \qquad (5)$$

wherein:

- h is the blood pool's height; and
- $P_t$ is the wet perimeter.

According to one or more embodiments, the blood's diffusion coefficient $D_{blood}$ may be measured experimentally. According to one or more embodiments, $D_{blood}$ may be measured for a plurality of predetermined environmental conditions or, for example, for a set of predetermined environmental conditions susceptible of representing the environmental conditions to which a blood pool may be commonly exposed.

According to the invention, the environmental conditions comprise at least the temperature and humidity to which the blood pool is exposed, and the nature of the substrate on which the substrate lies. The temperature and humidity may refer to the temperature and humidity in the direct surroundings of the blood pool, for example within a 3m radius from the blood pool. The nature of the substrate may comprise the composition of the substrate.

Determining the drying front of the blood pool at the given time t comprises determining the wet perimeter of the blood pool at the given time t. Such wet perimeter of the blood pool at the given time t is designated by reference sign $P_t$ in Figure 2 which illustrates a drying blood pool. At a given time t, the wet perimeter is the path surrounding the wet area of the blood pool. The wet area of the blood pool at the given time t is designated by reference sign $A_t$ in Figure 2 and is delimited by a wet perimeter $P_t$ at time t. The wet area $A_t$ corresponds to the remaining blood pool's liquid surface at time t, by opposition to the blood pool's dry surface at time t. By analogy, the wet area of the blood pool at time $t_0$ at which the blood pool initiated a drying process on the substrate is designated by reference sign $A_0$ in Figure 2 and is delimited by a wet perimeter $P_0$ at time $t_0$. The area $A_0$ is the maximum spreading area of the blood pool on the substrate.

According to one or more embodiments, determining the drying front of the blood pool at the given time t further comprises determining the wet area of the blood pool at the given time t, *i.e.,* $A_t$.

According to one or more embodiments, the method comprises taking at least one picture of the blood pool at a given time t, for example a plurality of pictures of the blood pool starting from the given time t. According to one or more embodiments, the method comprises taking one picture per 10 minutes for a predetermined period of time, such as for example for 1 hour. In this manner, the margin of error in dating the blood pool may thereby be lowered.

According to one or more embodiments, such at least one picture of the blood pool can be taken in several ways. For example, depending on the country, the forensic standards for taking a picture on a crime scene may vary, and the method according to the invention may be adapted accordingly. According to one or more embodiments, the at least one picture is taken on top of the blood pool, at a distance from the blood pool comprised between 0.1 m and 3 m, depending on the size of the blood pool. According to one or more embodiments, the at least one picture is taken on top of the pool, as perpendicularly as possible from the pool with a ruler beside.

According to one or more embodiments, the at least one picture may be taken with a conventional digital camera. A conventional digital camera may for example be integrated in a smartphone or a tablet.

According to one or more embodiments, the resolution of the at least one picture is more than 1000 pixels$^2$, for example more than 2000 pixels$^2$.

According to one or more embodiments, the determining of the drying front of the blood pool is performed via image processing of the at least one picture. For example, when an image processing software such as ImageJ or for example a Python code is used, a minimum resolution of 2000 pixels$^2$ may be advantageous in order to better focus on the blood pool only and an overexposure and/or a flash may result in a better contrast between the wet substrate and the dry substrate.

In the article Laan et al., Morphology of drying blood pools, Forensic Sci. Int. 267, pages 104-109, 2016, the authors have shown that blood pools' drying process can be separated into five main different drying stages: coagulation, gelation, rim desiccation, center desiccation, and final desiccation. According to one or more embodiments of the present disclosure, the step of taking at least one picture of the blood pool at a given time is performed either during the rim desiccation stage or during the center desiccation stage of the blood pool's drying process and, when the method comprises taking a plurality of pictures of the blood pool at a given time, in at least one of these stages. Contrary to the first drying stage, *i.e.,* coagulation, wherein the wet area at a given time t is equal or very close to the wet area at time

$t_0$, the wet area at a given time t during the rim desiccation or center desiccation stages is sufficiently different from the wet area at time $t_0$. Likewise, in these embodiments, the wet perimeter at time t is sufficiently different from the wet perimeter at time $t_0$. Also, in these embodiments, the wet area and the wet perimeter at a given time t are not equal to 0, while this is the case in the last drying stage, *i.e.,* in the final desiccation.

Thus, according to these embodiments, if the blood pool is in a coagulation stage, the at least one picture should be taken in a later stage, for example starting from the rim desiccation stage. If the blood pool is in a still later drying stage such as the final desiccation stage, the least one picture may be taken, but the wet perimeter and/or the wet area of the blood pool may not be determined anymore, since they are both equal to zero in such stage. However, in the majority of cases, the blood pools found on crime scenes have not yet reached this final stage of the blood pool's drying process.

According to one or more embodiments, the drying model comprises a function correlating the mass variation of the blood pool with time with the wet area of the blood pool at the given time t. According to one or more embodiments, such function comprises correlation coefficients which may be measured experimentally, for a plurality of predetermined environmental conditions or, for example, for a set of predetermined environmental conditions susceptible of representing the environmental conditions to which a blood pool may be commonly exposed.

According to one or more embodiments, the method further comprises determining the blood pool's height. According to one or more embodiments, determining the blood pool's height comprises the use of a statistical value of the blood pool's height. The statistical value may for example be an average value of blood pool's height measured for the same environmental conditions. According to one or more embodiments, a statistical value of the blood pool's height may be the average value of blood pool's height measured for a predetermined number of blood pools having the same area of the blood pool at a time at which the blood pool initiated a drying process on the substrate, and for the same environmental conditions. According to one or more embodiments, the predetermined number of blood pools is no less than 5, for example no less than 10, and may less than 20, and their mass may be comprised, for example, from 0.1 g to 50 g.

According to one or more embodiments, the function correlating the mass variation of the blood pool with time with the wet area of the blood pool at the given time t is expressed according to the following equation:

$$\frac{\delta m}{\delta t} = -\frac{\alpha\, m_0 \left[1 - \left(\frac{A_t}{A_0}\right)\right]^{\beta}}{\delta t} \qquad (6)$$

wherein:

- $\alpha$ and $\beta$ are correlation coefficients constant at the environmental conditions to which the blood pool is exposed at the given time t;
- $A_t$ is the wet area of the blood pool at the given time t;
- $\delta t$ is the time elapsed between the given time t and the initial time $t_0$;
- $\delta m$ is the mass variation of the blood pool during the time elapsed between the given time t and the initial time $t_0$;
- $m_0$ is the mass of the blood pool at the initial time $t_0$ and is expressed as according to the following equation:

$$m_0 = A_0 \rho h \qquad (7)$$

wherein:

- $\rho$ is the blood's volume weight.

According to one or more embodiments, the function correlating the mass variation of the blood pool with time with the wet area of the blood pool at the given time may be expressed according to the following equation:

$$\frac{m_t}{m_0} = 1 - \alpha \left[1 - \frac{A_t}{A_0}\right]^{\beta} \qquad (6')$$

wherein $m_t$ is the mass variation of the blood pool at the given time t.

This equation represents the variation of the normalized mass $m_t/m_0$ as a function of the normalized wet area ($A_t/A_0$), and involves correlation coefficients $\alpha$ and $\beta$.

As mentioned above, according to one or more embodiments, the function correlating the mass variation of the blood pool with time with the wet area of the blood pool at the given time t comprises correlation coefficients $\alpha$ and $\beta$ which may be measured experimentally, for a plurality of predetermined environmental conditions or, for example, for a set of predetermined environmental conditions susceptible of representing the environmental conditions to which a blood pool may be commonly exposed.

According to the invention, the sets of measured blood pool parameters including blood's diffusion coefficients $D_{blood}$ and blood pool's heights h, and which are associated with the sets of predetermined environmental and initial blood pool conditions comprised in the database as defined above with respect to one or more embodiments of the method of the first aspect, further comprise the correlation coefficients $\alpha$ and $\beta$.

According to one or more embodiments, the correlation coefficients $\alpha$ and $\beta$ may be measured for a predetermined number of blood pools having different masses. For example, the correlation coefficients $\alpha$ and $\beta$ may be determined out of a pool of at least 4 blood pools having different masses, for example a first blood pool weighing from 1g to 2g, a second blood pool weighing from 5g to 6g, a third blood pool weighing from 10g to 12g, and a fourth blood pool weighing from 30g to 35g. According to the invention, a fitting curve may be deduced from the superimposition of the predetermined number of corresponding variations of the normalized mass $m_t/m_0$ as functions of the normalized wet area $A_t/A_0$. Such fitting curve may thus provide the correlation coefficients $\alpha$ and $\beta$.

According to one or more embodiments, the time elapsed $\delta t$ between the given time t and the initial time $t_0$ is expressed according to the following equation:

$$\delta t \; = \; \frac{\alpha \, R k_B T^2 {A_0}^{1/2} h^{1/2} \rho [1 - \left(\frac{A_t}{A_0}\right)]^{\beta}}{\pi d^2 M {P_t}^{1/2} D_{blood} P_w P_a} \qquad (8)$$

The equations (1) and (6) are combined with each other into equation (8) by considering that the evaporation rate J* may be expressed as the variation of mass with time per surface unit according to equation (3).

Hence, as apparent from equation (8), the inventors have surprisingly managed to devise one equation correlating all drying parameters of the drying model according to one or more embodiments with the elapsed time $\delta t$. The inventors have demonstrated that despite the complexity of establishing a drying model taking account of the variability of all parameters at stake during the blood pools' drying process, a reliable and simple drying model exists and may be used to develop a corresponding method for dating blood pools, in a fast, reliable, and accurate manner. According to one or more embodiments, the drying model may take account of the blood pool's size and/or shape, which are reflected by mathematical equations used in the modeling. For example, when one considers equation (8), correlation coefficients $\alpha$ and $\beta$ may take into account the size of the blood pool. The dimensionless factor L* takes account of the shape of the blood pool. Indeed, L*, which is directly related to the drying front, for example according to equation (5), correlates the shape properties of the blood pool with the wet perimeter.

According to one or more embodiments, the blood's volume weight is substantially comprised between 1.040 g/mL and 1.066 g/mL.

According to one or more embodiments, the method comprises a step of determining the atmospheric pressure. According to one or more embodiments, the atmospheric pressure is substantially equal to $1.01325.10^5$ Pa.

According to one or more embodiments, the saturation vapor pressure of water at the surface of the blood pool is deduced from determination of the temperature and humidity to which the blood pool is exposed at the given time t.

According to one or more embodiments, the molecular diameter of water molecules is substantially equal to 0.343 nm.

According to one or more embodiments, determining the environmental conditions to which a blood pool is exposed may be done by measuring the environmental conditions, directly and/or indirectly, on the crime scene. According to one or more embodiments, requesting the measurement of environmental conditions may for example be envisaged. For example, the method may comprise sending the GPS location of the blood pool to a weather forecast virtual platform and obtaining the measured data sent back by the weather forecast virtual platform. Such an indirect measurement may be beneficial when the blood pool has been drying outdoors, since environmental parameters such as the temperature and humidity may sensibly vary with time. A liability level of the recorded values can then be assessed, depending on the available data.

Indoors, in contrast, the environment of the blood pool is expected to be stable in the majority of cases, and a standard ambient temperature, for example of 20°C, may be used. Alternatively, a direct measuring of the environmental parameters may be performed.

According to one or more embodiments, the substrate on which the blood pool lies may be porous. According to one or more embodiments, the substrate may be non-porous, *i.e.*, so that the blood may remain substantially stagnant prior to drying. According to one or more embodiments, the substrate on which the blood pool lies may be selected in the group comprising tiling, linoleum, varnished parquet, unvarnished parquet, glass, paper, wood (for example oak), fabric, asphalt, carpet, grass.

According to one or more embodiments, the blood comprised in the blood pool has a hematocrit level Ht comprised between 35% and 55%, for example between 40% and 50%. The hematocrit level Ht is comprised between 40.7% and 50.3% for men and between 36.1% and 44.3% for women. The inventors have shown that the hematocrit level does not have a major influence on the blood pool's drying process. However, according to one or more embodiments, the method may be specifically adapted to men or women. According to one or more embodiments, the method comprises the use of two databases, one male database specifically adapted to men and one female database specifically adapted to women. For example, the method according to one or more embodiments may include a step of recording the sex of the blood giver. This will orient the method towards the use of a corresponding male or female database.

Hence, according to one or more embodiments of the method, the method envisages the determination of certain parameters, such as for example $P_t$, $A_t$ and/or $A_0$, on the basis of the image processing of at least one blood pool's picture, and the determination of parameters related to the environment of the blood pool, such as temperature, humidity, and to the nature of the substrate. All other parameters may be collected and saved beforehand in a database. Such a dating method allows a very precise and reliable dating of the blood pool. The inventors have indeed realized that such a method is particularly adapted to the dating of blood pools and shows excellent results in dating blood pools. For example, an error margin of less than 4% could be obtained in dating blood pools that had been drying for more than 8h, by using the method according to one or more embodiments of the present disclosure. Such method for dating blood pools according to the invention provides a low-cost, rapid, easy-to-use and truly portable alternative to more complicated analysis using specialized and highly expensive equipment, e.g. spectroscopy and/or HPLC.

According to one or more embodiments, the method according to the invention comprises the repetition of at least the following steps a predetermined number of times:

- determining, based on at least one picture of a blood pool lying on a substrate taken at a given time t, the drying front of the blood pool at the given time t; and
- determining, based on a drying model and the determined drying front of the blood pool at the given time (t), the time elapsed between the given time t and a time $t_0$ at which the blood pool initiated a drying process on the substrate.

Such repetition may for example occur at predetermined time intervals during a predetermined period.

In this manner, an average value of the elapsed time between the given time t and a time $t_0$ at which the blood pool initiated a drying process on the substrate may be obtained.

According to a second aspect, the present disclosure is directed to a system for dating a blood pool lying on a substrate, comprising:

- means for determining, based on at least one picture of a blood pool lying on a substrate taken at a given time t, the drying front of the blood pool at the given time t; and
- means for determining, based on a drying model and on the determined drying front of the blood pool at the given time t, the time elapsed between the given time t and an initial time $t_0$ at which the blood pool initiated a drying process on the substrate,

wherein the means for determining the drying front of the blood pool at the given time t determines the wet perimeter $P_t$ of the blood pool at the given time t, and
wherein the means for determining the time elapsed between the given time t and the initial time $t_0$ correlates the time elapsed with a blood's diffusion coefficient $D_{blood}$ measured for a plurality of predetermined environmental conditions and a plurality of initial blood pool conditions.

According to one or more embodiments, the means for determining the drying front of the blood pool at the given time t comprises at least one image processing software that performs an image processing of the at least one picture of the blood pool. Such at least one image processing software may for example be installed on a smartphone or tablet. According to one or more embodiments, the at least one image processing software is selected in the group comprising ImageJ, a Matlab code, a Python code.

According to one or more embodiments, the means for determining the time elapsed between the given time t and the initial time $t_0$ comprises a calculation software.

According to one or more embodiments, the system further includes a database comprising sets of predetermined environmental and initial blood pool conditions including initial areas ($A_0$), the sets of predetermined environmental and initial blood pool conditions being associated with corresponding sets of measured blood pool parameters including blood's diffusion coefficients ($D_{blood}$) and blood pool's heights (h).

According to one or more embodiments, the system according to the second aspect may be used to implement the method according to the first aspect. Such system may be a mobile system offering an easy-to-use and portable means for dating blood pools, wherein no training is necessary, such as for example a smartphone wherein a dating software is installed that performs the steps of the method for dating blood pools. Such dating software may provide an accurate age estimate by taking account of a user's inputs, such as pictures of the blood pool or determined environmental and initial blood pool conditions impacting the drying of blood pools.

According to one or more embodiments, the system further comprises a camera for taking the at least one picture of the blood pool lying on a substrate. According to one or more embodiments, the system comprises a conventional digital camera. Such conventional digital camera may for example be integrated in a smartphone or a tablet. The model of the camera may have an influence on, for example, the color rendering of the picture of the blood pool. Hence, according to one or more embodiments, measures may be taken so that parameters of the smartphone are set so that they may be easily adapted to different forensic standards, depending on the geographical zone of a crime scene.

According to one or more embodiments, the system further comprises a thermometer and a hygrometer for determining the temperature and, respectively, the humidity to which the blood pool is exposed.

According to one aspect, the present disclosure concerns a method for dating a blood pool lying on a substrate, comprising:

- determining, based on at least one picture of a blood pool lying on a substrate taken at a given time, the drying front of the blood pool at the given time; and
- determining, based on the determined drying front of the blood pool at the given time and a drying model, the time elapsed between the given time and a time at which the blood pool initiated a drying process on the substrate,

wherein the drying model comprises a function correlating the time elapsed between the given time (t) and a time ($t_0$) at which the blood pool initiated a drying process on the substrate with a blood's diffusion coefficient, the blood's diffusion coefficient having a substantially constant value during the time elapsed between the given time (t) and a time ($t_0$) at which the blood pool initiated a drying process on the substrate for the same environmental conditions, wherein the blood's diffusion coefficient is expressed according to the following equation:

$$D_{blood} = K_i L_k L^{*0.5} \qquad (1)$$

wherein:

- $D_{blood}$ is the blood's diffusion coefficient;
- $K_i$ is a coefficient of transfer from the liquid state into the gaseous state and is expressed according to the following equation:

$$K_i = J^* \frac{RT}{MP_w} \qquad (2)$$

wherein:

- M is the molecular weight of water;
- $P_w$ is the saturation vapor pressure of water at the surface of the blood pool;
- R is the ideal gas constant;
- T is the temperature of the environment to which the blood pool is exposed; and
- J* is the evaporation rate of the blood pool and is expressed according to the following equation:

$$J^* = \frac{\delta m}{\delta t} \times \frac{1}{A_0} \qquad (3)$$

wherein:

- $\delta m/\delta t$ is the mass variation of the blood pool with time; and
- $A_0$ is the area of the blood pool at a time ($t_0$) at which the blood pool initiated a drying process on the substrate;

- $L_k$ is the Knudsen layer and is expressed according to the following equation:

$$L_k = \frac{k_B T}{\pi d^2 P_a} \quad (4)$$

wherein:

- $k_B$ is Boltzmann's constant;
- d is the molecular diameter of water molecules; and
- $P_a$ is the atmospheric pressure;

- L* is a shape factor and is expressed according to the following equation:

$$L^* = \frac{A_0}{P_t h} \quad (5)$$

wherein:

- h is the blood pool's height; and
- $P_t$ is the wet perimeter.

According to one or more embodiments, determining the drying front of the blood pool at the given time t comprises determining the wet perimeter of the blood pool at the given time t. According to one or more embodiments of the method according to the embodiments described at page 15, line 14, determining the drying front of the blood pool at the given time t further comprises determining the wet area of the blood pool at the given time t, *i.e.,* $A_t$. According to one or more embodiments, the drying model comprises a function correlating the mass variation of the blood pool with time with the wet area of the blood pool at the given time t. According to one or more embodiments, the method further comprises determining the area of the blood pool at a time at which the blood pool initiated a drying process on the substrate and the environmental conditions to which the blood pool is exposed at the given time.

According to one or more embodiments of the method according to the embodiments described at page 15, line 19 and according to the embodiments described at page 15, line 21 when they also include the features of the embodiments described at page 15, line 19, the function correlating the mass variation of the blood pool with time with the wet area of the blood pool at the given time (t) is expressed according to the following equation:

$$\frac{\delta m}{\delta t} = -\frac{\alpha\, m_0 \left[1 - \left(\frac{A_t}{A_0}\right)\right]^{\beta}}{\delta t} \quad (6)$$

wherein:

- $\alpha$ and $\beta$ are correlation coefficients constant at the environmental conditions to which the blood pool is exposed at the given time t;
- $A_t$ is the wet area of the blood pool at the given time t;
- $\delta t$ is the time elapsed between the given time t and a time $t_0$ at which the blood pool initiated a drying process on the substrate;
- $\delta m$ is the mass variation of the blood pool during the time elapsed between the given time t and a time $t_0$ at which the blood pool initiated a drying process on the substrate;
- $m_0$ is the mass of the blood pool at a time $t_0$ at which the blood pool initiated a drying process on the substrate and is expressed as according to the following equation:

$$m_0 = A_0 \rho h \quad (7)$$

wherein:

- $\rho$ is the blood's volume weight.

According to one or more embodiments of the method according to the embodiments described at page 15, line 24, the time elapsed δt between the given time t and the time $t_0$ at which the blood pool initiated a drying process on the substrate is expressed according to the following equation:

$$\delta t \; = \; \frac{\alpha \, R k_B T^2 {A_0}^{1/2} h^{1/2} \rho [1 - \left(\frac{A_t}{A_0}\right)]^\beta}{\pi d^2 M {P_t}^{1/2} D_{blood} P_w P_a} \qquad (8)$$

According to one or more embodiments of the method according to the embodiments described at page 15, line 24 or according to the embodiments described at page 16, line 15, the method further comprises providing a database comprising sets of measured blood pool parameters at different conditions, wherein each set of measured blood pool parameters comprises the blood's diffusion coefficient, the blood pool's height, and the correlation coefficients, wherein the different conditions comprise predetermined environmental and initial blood pool conditions impacting the drying of blood pools. According to one or more embodiments of the method according to the embodiments described at page 16, line 20, the predetermined environmental conditions impacting the drying of blood pools comprise at least the temperature and humidity of the environment to which the blood pool is exposed, and the nature of the substrate on which the blood pool lies. According to one or more embodiments of the method according to the embodiments described at page 16, line 20 or according to the embodiments described at page 16, line 26, the predetermined initial blood pool conditions impacting the drying of blood pools comprise the area of the blood pool at a time at which the blood pool initiated a drying process on the substrate. According to one or more embodiments of the method according to the embodiments described at page 16, line 30 when they include the features of the embodiments described at page 16, line 20 when they include the features of the embodiments described at page 15, line 24, when they include the features of the embodiments described at page 15, line 21, the method providing the database further comprises the steps of comparing the determined environmental conditions to which the blood pool is exposed at the given time t and the determined area of the blood pool at a time $t_0$ at which the blood pool initiated a drying process on the substrate, with the sets of predetermined environmental and initial blood pool conditions impacting the drying of blood pools comprised in the database.

According to one or more embodiments, the method comprises the repetition of at least the following steps a predetermined number of times: determining, based on at least one picture of a blood pool lying on a substrate taken at a given time t, the drying front of the blood pool at the given time t; and determining, based on a drying model and the determined drying front of the blood pool at the given time (t), the time elapsed between the given time t and a time $t_0$ at which the blood pool initiated a drying process on the substrate. Such repetition may for example occur at predetermined time intervals during a predetermined period. In this manner, an average value of the elapsed time between the given time t and a time $t_0$ at which the blood pool initiated a drying process on the substrate may be obtained.

According to one aspect, the present disclosure is directed to a system for dating a blood pool lying on a substrate, comprising:

- means for determining, based on at least one picture of a blood pool lying on a substrate taken at a given time t, the drying front of the blood pool at the given time t; and
- means for determining, based on a drying model and the determined drying front of the blood pool at the given time t, the time elapsed between the given time t and a time $t_0$ at which the blood pool initiated a drying process on the substrate,

wherein the drying model comprises a function correlating the time elapsed between the given time (t) and a time ($t_0$) at which the blood pool initiated a drying process on the substrate with a blood's diffusion coefficient, the blood's diffusion coefficient having a substantially constant value during the time elapsed between the given time (t) and a time ($t_0$) at which the blood pool initiated a drying process on the substrate for the same environmental conditions,

wherein the blood's diffusion coefficient is expressed according to the following equation:

$$D_{blood} = K_i L_k L^{*0.5} \qquad (1)$$

wherein:

- $D_{blood}$ is the blood's diffusion coefficient;
- $K_i$ is a coefficient of transfer from the liquid state into the gaseous state and is expressed according to the following equation:

$$K_i = J^* \frac{RT}{MP_w} \qquad (2)$$

wherein:

- M is the molecular weight of water;
- $P_w$ is the saturation vapor pressure of water at the surface of the blood pool;
- R is the ideal gas constant;
- T is the temperature of the environment to which the blood pool is exposed; and
- J* is the evaporation rate of the blood pool and is expressed according to the following equation:

$$J^* = \frac{\delta m}{\delta t} \times \frac{1}{A_0} \qquad (3)$$

wherein:

- $\delta m/\delta t$ is the mass variation of the blood pool with time; and
- $A_0$ is the area of the blood pool at a time ($t_0$) at which the blood pool initiated a drying process on the substrate;

- $L_k$ is the Knudsen layer and is expressed according to the following equation:

$$L_k = \frac{k_B T}{\pi d^2 P_a} \qquad (4)$$

wherein:

- $k_B$ is Boltzmann's constant;
- d is the molecular diameter of water molecules; and
- $P_a$ is the atmospheric pressure;

- L* is a shape factor and is expressed according to the following equation:

$$L^* = \frac{A_0}{P_t h} \qquad (5)$$

wherein:

- h is the blood pool's height; and
- $P_t$ is the wet perimeter.

According to one or more embodiments of the system according to the aspect described at page 17, line 22, the means for determining, based on at least one picture of a blood pool lying on a substrate taken at a given time t, the drying front of the blood pool at the given time t, comprises at least one image processing software that performs an image processing of the at least one picture of the blood pool. Such at least one image processing software may for example be installed on a smartphone or tablet. According to one or more embodiments, the at least one image processing software is selected in the group comprising ImageJ, a Matlab code, a Python code. According to one or more embodiments, the means for determining, based on a drying model and the determined drying front of the blood pool at the given time t, the time elapsed between the given time t and a time $t_0$ at which the blood pool initiated a drying process on the substrate comprises a calculation software. According to one or more embodiments, the system further comprises a database comprising sets of measured blood pool parameters at different conditions, wherein each set of measured blood pool parameters comprises a blood's diffusion coefficient, a blood pool's height, and parameters correlating the mass variation of the blood pool with time with the wet area of the blood pool at the given time t, and wherein the different conditions comprise predetermined environmental and initial blood pool conditions impacting the drying of blood pools.
According to one or more embodiments, the system further comprises a camera for taking the at least one picture of the blood pool lying on a substrate. According to one or more embodiments, the system comprises a conventional digital camera. According to one or more embodiments, the system further comprises a thermometer and a hygrometer for determining the temperature and, respectively, the humidity to which the blood pool is exposed.

The embodiments described above are not exhaustive. In particular, it is understood that additional embodiments can be considered on the basis of different combinations of the explicitly described embodiments. Unless otherwise specified in the present disclosure, it will be apparent to the skilled person that all the embodiments described above can be combined together. For example, unless otherwise specified, all features of the embodiments described above, whether they refer to the method or the system for dating blood pools, can be combined with or replaced by other features from other embodiments.

Brief description of the drawings

The present disclosure will be better understood and other advantages and embodiments will become clear on reading the description that follows, given purely by way of indication and in no way limiting, and by referring to the appended figures, in which:

- FIG. 1A is a transversal scheme illustrating a blood drop.

- FIG. 1B is a transversal scheme illustrating a blood pool.

- FIG. 1C is a transversal scheme illustrating an infinite stretch of blood.

- FIG. 2 is a top view of a sample of drying blood pool in which parameters such as $A_0$, $P_0$, $A_t$ and $P_t$ are shown.

- FIG. 3 is a simplified scheme illustrating an example of method for dating blood pools lying on a substrate according to one or more embodiments of the present disclosure.

- FIG. 4 is a diagram correlating $K_i L_k L^{*0.5}$ with the percentage of water left in different blood pools, drying at 20% humidity, T = 23°C $\pm$ 1°C, on the same surface, a tile. The experimentally measured blood's diffusion coefficient $D_{blood}$ corresponds to the plateau value equal to $10^{-9} m^2.s^{-1}$.

- FIG. 5 is a diagram correlating the normalized mass with the normalized wet area for different blood pools, drying at 20% humidity, T = 23°C $\pm$ 1°C, on the same surface, a tile.

- FIG. 6 is a diagram correlating $K_i L_k L^{*0.5}$ with the percentage of water left in blood pools having the same mass (4,83 $\pm$ 0,026 g) and drying at different humidities (30 %, 40 %, 50 %), at the same temperature T = 21°C $\pm$ 1°C, on different surfaces (varnished or unvarnished parquet). Different values blood's diffusion coefficient $D_{blood}$ corresponding to plateau values are obtained.

Detailed description of embodiments

Embodiments of the present disclosure will now be described in detail with reference to the accompanying figures. In the following detailed description of embodiments of the present disclosure, numerous specific details are set forth in order to provide a more thorough understanding of the present disclosure. However, it will be apparent to one of ordinary skill in the art that the present disclosure may be practiced without these specific details. In other instances, well-known features have not been described in detail to avoid unnecessarily complicating the description.

The following description provides a non-limiting example of a system for dating a blood pool lying on a substrate, according to one or more embodiment of the present disclosure.

The following description also provides non-limiting examples of a method for dating a blood pool lying on a substrate according to one or more embodiments of the present disclosure.

Example of a system for dating a blood pool lying on a substrate

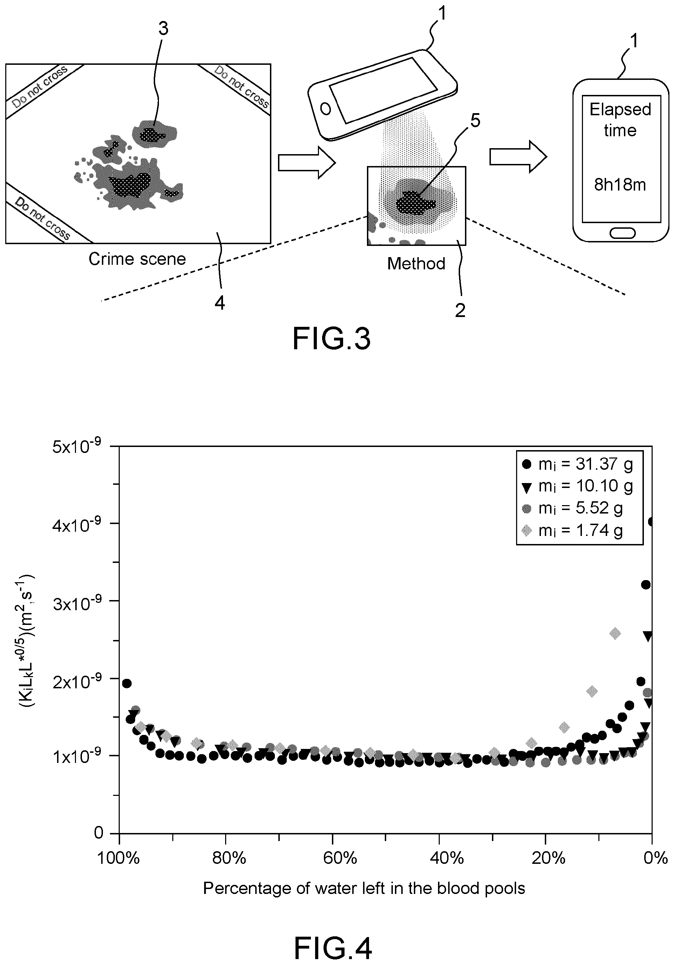

An example of system for dating a blood pool lying on a substrate is shown in Figure 3. The system comprises a smart phone 1 comprising a conventional camera (not shown) for taking pictures and an image processing software for determining, based on at least one picture 2 of a blood pool 3 lying on a substrate 4 taken at a given time t, the drying front 5 of the blood pool 3 at the given time t. The system further comprises a calculation software for determining, based on the determined drying front of the blood pool at the given time t and a drying model, the time elapsed between the given time t and a time $t_0$ at which the blood pool initiated a drying process on the substrate.

In the present example, the image processing software is imageJ. However, other image processing softwares may be run in the smart phone. Also, according to other embodiments, the image processing software may be run in other devices.

The image processing software may be integrated within a dating software.

The imageJ software may be used to determine the area of the blood pool at a time $t_0$ at which the blood pool initiated a drying process on the substrate from a picture of the blood pool. The type of image may first be changed from RGB color type to 8 or 16-bit type image, the black and white threshold may then be manually adjusted in order to define the contours of the blood pool. To measure the area of the blood pool, an "analyze particles" function may used. The minimum pixel size may be set to 2000 pixels$^2$ in order to focus on the blood pool only. Holes may be included in order to exclude reflects that may be present on the picture, and the outlines of the measured area may be displayed with the results in order to verify the accuracy of the area's measurement.

The calculation software may be integrated within a dating software, which may be the same as the above-mentioned dating software comprising the above-mentioned image processing software.

In the present example, the dating software is configured to request the measurement of two environmental conditions, namely temperature and humidity, to a weather forecast virtual platform and to obtain the measurement by sending the GPS location to the weather forecast virtual platform.

In the present example, the dating software is also configured to ask a user to upload the at least one picture taken by the camera and to fill out blank fields corresponding to a third environmental condition, namely the nature of the substrate.

In this example of system, the system further comprises a database including sets of measured blood pool parameters at different conditions. Each set of measured blood pool parameters comprises a blood's diffusion coefficient $D_{blood}$, a blood pool's height h, and parameters correlating the mass variation of the blood pool with time with the wet area of the blood pool at the given time t. The different conditions comprise predetermined environmental and initial blood pool conditions impacting the drying of blood pools. The predetermined environmental conditions comprise the temperature and humidity to which the blood pool is exposed and the nature of the substrate on which the substrate lies. The predetermined initial blood pool conditions comprise the area of the blood pool at a time $t_0$ at which the blood pool initiated a drying process on the substrate.

As mentioned above, this example of system for dating blood pool according to the embodiments of the disclosure comprises a smartphone including a conventional camera, which can be used to take one or more pictures of a blood pool lying on a substrate. However, further configurations of the system may be envisaged, in which the system also comprises, for example, a thermometer and/or a hygrometer.

Example of a method for dating a blood pool lying on a substrate

The following describes an example of a computer-implemented method for dating a blood pool lying on a substrate according to embodiments of the present disclosure.

The method for dating a blood pool lying on a substrate comprises determining, based on at least one picture of a blood pool lying on a substrate taken at a given time t, the drying front of the blood pool at the given time t, and determining, based on the determined drying front of the blood pool at the given time t and on a drying model, the time elapsed between the given time t and an initial time $t_0$ at which the blood pool initiated a drying process on the substrate.

The drying model comprises a function correlating the time elapsed between the given time t and a time $t_0$ at which the blood pool initiated a drying process on the substrate with a blood's diffusion coefficient, the blood's diffusion coefficient having a substantially constant value during the time elapsed between the given time t and a time $t_0$ at which the blood pool initiated a drying process on the substrate for the same environmental conditions. The drying model comprises a blood's diffusion coefficient $D_{blood}$. Such blood's diffusion coefficient is expressed according to mathematical equation (1). In this example, the blood's diffusion coefficient was measured experimentally for a set of predetermined environmental conditions susceptible of representing the environmental conditions to which a blood pool may be commonly exposed. Hence, such blood's diffusion coefficient was available beforehand to users of the system, *i.e.*, before dating the blood pool, and was stored in the above-mentioned database.

In this example of method, determining the drying front comprised determining the wet perimeter $P_t$ and the

wet area $A_t$. Both were determined by image processing a picture of the blood pool taken at the given time t. In this example of method according to the invention, numerical values of 0.16 m and 0.024 m$^2$ were determined for the wet perimeter and the wet area, respectively.

The drying model of this example of method further comprises a function correlating the mass variation of the blood pool with time with the wet area of the blood pool at the given time t. The function is expressed according to equation (6').

The evaporations of 4 blood pools having different masses (*i.e.*, 1.75 g, 5.52 g, 10.10 g, 31.37 g) were plotted with time at 20% humidity, T = 23°C $\pm$ 1°C, on the same surface, a tile. For each of the 4 blood pools, $K_i.L_k.L^{*0,5}$ of equation (1) was plotted with time which yielded a common plateau (see Figure 4), yielding a substantially constant value of 10$^{-9}$ m$^2$.s$^{-1}$ for the blood's diffusion coefficient $D_{blood}$.

In this example of method according to embodiments of the disclosure, the area $A_0$ of the blood pool at a time $t_0$ at which the blood pool initiated a drying process on the substrate and the environmental conditions to which the blood pool was exposed at the given time t, were determined. The determination of the area $A_0$ was performed via image processing the picture of the blood pool taken at the given time t.

The method further comprised determining the blood pool's height. For this purpose, a statistical value of the blood pool's, *i.e.,* the average value of blood pool's height measured for 30 blood pools having the same area of the blood pool at a time $t_0$ at which the blood pool initiated a drying process on the substrate, was measured, for sets of predetermined environmental conditions. For example, at a temperature of 23 $\pm$ 1°C and a humidity of 20%, for a white tile as substrate, such measurement provides 1.44 mm ($\pm$ 0.19 mm) as the statistical value of the blood pool's height.

In this example of method, the function correlating the mass variation of the blood pool with time with the wet area of the blood pool at the given time t was expressed according to the following equation:

$$\frac{m_t}{m_0} = 1 - \alpha \left[ 1 - \frac{A_t}{A_0} \right]^{\beta} \qquad (6')$$

This equation represents the variation of the normalized mass $m_t/m_0$ as a function of the normalized wet area $A_t/A_0$, and involves correlation coefficients $\alpha$ and $\beta$.

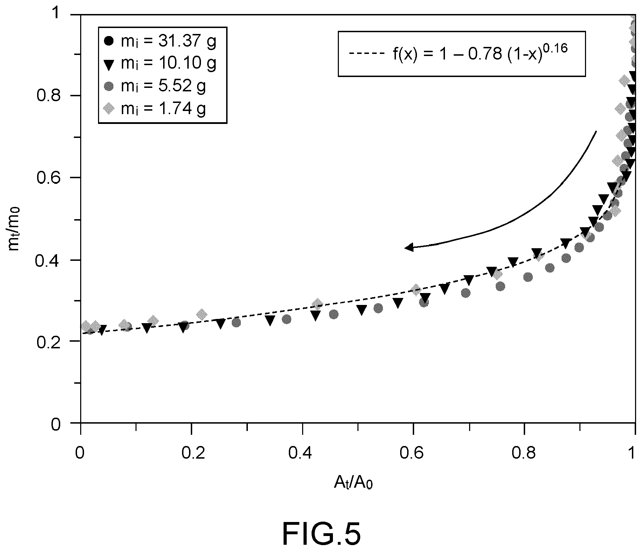

The normalized mass of the blood pool was plotted with the normalized wet area of the blood pool at the given time t for 3 blood pools having different masses (*i.e.*, 1.74 g, 5.52 g, 10.10 g) (see Figure 5). A fitting curve was deduced from the superimposition of the 3 corresponding variations of the normalized mass $m_t/m_0$ as function of the normalized wet area $A_t/A_0$. Such fitting curve provided the correlation coefficients $\alpha$ and $\beta$.

The method allowed to determine the time elapsed $\delta t$ between the given time t and a time $t_0$ at which the blood pool initiated a drying process on the substrate. The time elapsed $\delta t$ was calculated according to equation (8).

As numerical values, the blood's volume weight $\rho$ was considered to be equal to 1.060 g/mL, the atmospheric pressure $P_a$ was considered to be equal to 1.01325.10$^5$ Pa, the saturation vapor pressure of water $P_w$ at the surface of the blood pool was obtained from standard charts, and the molecular diameter of water molecules was considered to be equal 0.343 nm.

Validation test of the method for dating a blood pool lying on a substrate

Blood was spilled on a white tile at a time $t_0$, *i.e.,* at 10.17 am, thereby forming a blood pool initiating a drying process at time $t_0$. A picture of the blood pool was then taken by a user via a system for dating blood pool according to the embodiments of the present disclosure while drying on the white tile, at a given time t, which was 6.54 pm, on the same day.

The user then proceeded to date the blood pool as follows.

Environmental conditions were determined. First, the GPS location of the blood pool was sent to a weather forecast virtual platform, and the temperature (23°C) and humidity (20%) were obtained in this manner. At this point, the user opened the dating software on the smartphone, and was asked by the dating software to upload the picture taken at 6.54 pm and to fill out blank fields corresponding to environmental parameters, *i.e.*, the nature of the substrate, *i.e.*, the tile. The user filled out the blank field. Thanks to the inclusion of the software imageJ within the dating software, the dating software then ran the image processing of the picture taken at time t and extracted $A_t$, $P_t$, $A_0$ of the blood pool corresponding to the following values: $A_0$ = 3.47E-03 m$^2$; $P_t$ = 230E-03 m; $A_t$ = 3,17E-03 m$^2$.

The calculation software compared these determined environmental conditions and the determined $A_0$ with the sets of predetermined environmental and initial blood pool conditions impacting the drying of blood pools comprised in the database.

The calculation software, based on this comparison, determined numerical values associated to the determined environmental conditions for the correlation coefficients $\alpha$ and $\beta$, equal to 0.78 and 0.16, respectively, for the blood's diffusion coefficient $D_{blood}$, equal to $10^{-9}$ m$^2$.s$^{-1}$, and for the blood pool's height, equal to 1.44 mm.

For these values, the calculated elapsed time $\delta t$ was 498 min (8h18min), which turned out to be 19 min less than the exact measured elapsed time between time t and time $t_0$ at which the blood pool was spilled on the white tile and initiated a drying process. This corresponded to a margin of error for the calculated elapsed time of less than 4 % of the exact elapsed time.

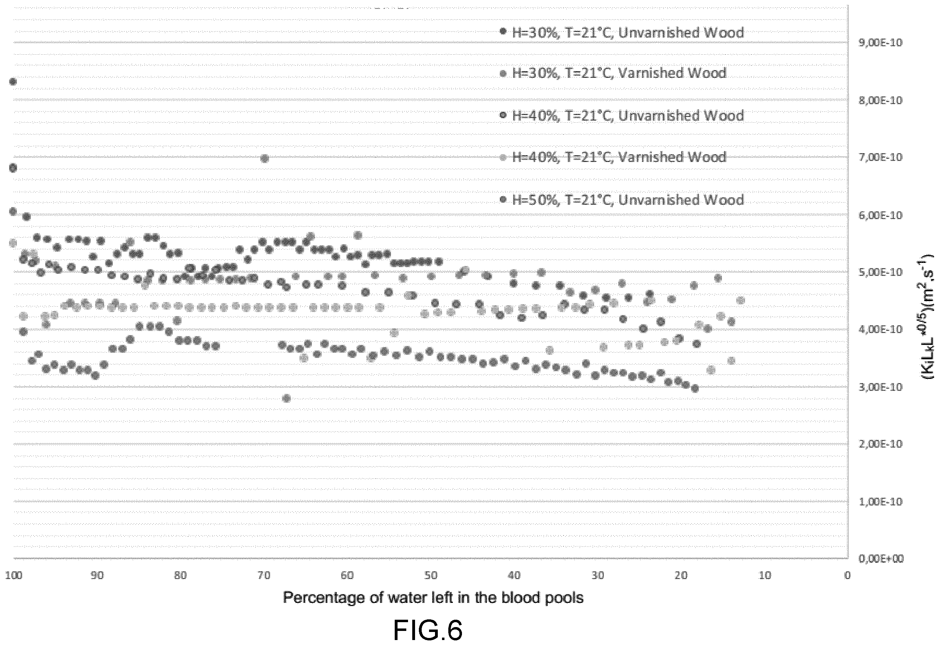

In this example of the method, the picture of the blood pool was taken only once. However, the taking of a predetermined number of pictures of the blood pool could have been envisaged in order to obtain an average value of the elapsed time $\delta t$. For example, in one or more embodiments of the present invention, the taking of a predetermined number of pictures can be done. Each repetition of such step can be followed by the repetition of the calculation of said elapsed time, thereby leading to the calculation of the elapsed time a predetermined number of times. At that point, an average value of the elapsed time may be obtained. According to one or more embodiments, such repetitions can be performed in an automated way, at a predetermined time interval, e.g. 1 picture being taken per minute during 10 minutes. In the above-detailed description, only one set of predetermined environmental and initial blood pool conditions is shown, which is associated with the value of $D_{blood}$ measured in FIG.4, *i.e.,* for a blood pool drying at 20% humidity, T = 23°C $\pm$ 1°C, on the same surface, a tile. However, the above-mentioned database, which comprises sets of predetermined environmental and initial blood pool conditions impacting the drying of blood pools associated with corresponding sets of measured blood pool parameters including blood's diffusion coefficients ($D_{blood}$) and blood pool's heights (h), may contain many more sets. FIG. 6 illustrates the interest of providing a rich database in the method according to one or more embodiments of the present disclosure. FIG. 6 is a diagram correlating $K_iL_kL^{*0.5}$ with the percentage of water left in blood pools having the same mass (4,83 $\pm$ 0,026 g) and drying at different humidities (30 %, 40 %, 50 %), at the same temperature T = 21°C $\pm$ 1°C, on different substrates 'surfaces (varnished or unvarnished parquet). The different values of blood's diffusion coefficients $D_{blood}$ corresponding to the plateau values observed in FIG.6 are presented in Table 1.

Table 1

| T(°C) | H(%) | Substrate | $D_{blood}$ (m$^2$.s$^{-1}$) |
|---|---|---|---|
| 21 | 30 | Unvarnished Parquet | $5.3 \times 10^{-10}$ |
| 21 | 30 | Varnished Parquet | $4.9 \times 10^{-10}$ |
| 21 | 40 | Unvarnished Parquet | $4.8 \times 10^{-10}$ |
| 21 | 40 | Varnished Parquet | $4.3 \times 10^{-10}$ |
| 21 | 50 | Unvarnished Parquet | $3.6 \times 10^{-10}$ |

**Claims**

1. Computer-implemented method for dating a blood pool lying on a substrate, comprising:

   - determining, based on at least one picture of a blood pool lying on a substrate taken at a given time (t), the drying front of the blood pool at the given time (t),
   - determining, based on the determined drying front of the blood pool at the given time (t), and on a drying model, the time elapsed between the given time (t) and an initial time ($t_0$) at which the blood pool initiated a drying process on the substrate,
   wherein determining the drying front of the blood pool at the given time (t) comprises determining the wet perimeter (Pt) of the blood pool at the given time (t), and
   wherein the drying model correlates the time elapsed with a blood's diffusion coefficient ($D_{blood}$) measured for a plurality of predetermined environmental conditions and a plurality of initial blood pool conditions; and
   wherein the time elapsed between the given time (t) and the initial time ($t_0$) is determined by:

   - providing a database comprising sets of predetermined environmental conditions comprising at least the temperature and humidity of the environment to which a blood pool is exposed, and the nature of the substrate on which a blood pool lies, and initial blood pool conditions including initial areas ($A_0$), the sets of predetermined environmental and initial blood pool conditions being associated with corresponding sets of measured blood pool parameters including blood's diffusion coefficients ($D_{blood}$), blood pool's heights (h), and correlation coefficients $\alpha$ and $\beta$,

wherein the measured blood pool parameters of each set of measured blood pool parameters are measured at the environmental and initial blood pool conditions of the associated set of predetermined environmental and initial blood pool conditions, and

wherein the correlation coefficients $\alpha$ and $\beta$ are measured for a predetermined number of blood pools having different masses, and provided by a fitting curve deduced from the superimposition of a predetermined number of corresponding variations of a normalized mass ($m_t/m_0$) as functions of a normalized wet area ($A_t/A_0$), wherein $m_t$ is the mass variation of the blood pool at the given time t, and $m_0$ is the mass of the blood pool at the initial time $t_0$,

- determining the environmental conditions comprising the temperature and humidity of the environment to which the blood pool is exposed at the given time (t) and the nature of the substrate on which the blood pool lies, and an initial area ($A_0$) of the blood pool corresponding to the area of the blood pool at the initial time ($t_0$),
- comparing the determined environmental conditions and the determined initial area ($A_0$) with the sets of predetermined environmental and initial blood pool conditions, and
- determining a set of measured blood pool parameters comprising the blood's diffusion coefficient ($D_{blood}$), the blood pool's height (h) and the correlation coefficients $\alpha$ and $\beta$, which are associated with the set of predetermined environmental and initial blood pool conditions identical to the determined environmental conditions and the determined initial area ($A_0$).

2. The computer-implemented method of claim 1, wherein the drying model comprises a function correlating the time elapsed between the given time (t) and the initial time ($t_0$) with the blood's diffusion coefficient ($D_{blood}$), the blood's diffusion coefficient having a substantially constant value during the time elapsed between the given time (t) and the initial time ($t_0$) for the same environmental conditions,

wherein the blood's diffusion coefficient is expressed according to the following equation:

$$D_{blood} = K_i L_k L^{*0.5} \qquad (1)$$

wherein:

- $D_{blood}$ is the blood's diffusion coefficient;
- $K_i$ is a coefficient of transfer from the liquid state into the gaseous state and is expressed according to the following equation:

$$K_i = J^* \frac{RT}{MP_w} \qquad (2)$$

wherein:

- M is the molecular weight of water;
- $P_w$ is the saturation vapor pressure of water at the surface of the blood pool;
- R is the ideal gas constant;
- T is the temperature of the environment to which the blood pool is exposed; and
- J* is the evaporation rate of the blood pool and is expressed according to the following equation:

$$J^* = \frac{\delta m}{\delta t} \times \frac{1}{A_0} \qquad (3)$$

wherein:

- $\delta m/\delta t$ is the mass variation of the blood pool with time; and
- $A_0$ is the area of the blood pool at the initial time ($t_0$);

- $L_k$ is the Knudsen layer and is expressed according to the following equation:

$$L_k = \frac{k_B T}{\pi d^2 P_a} \qquad (4)$$

wherein:

- $k_B$ is Boltzmann's constant;
- d is the molecular diameter of water molecules; and
- $P_a$ is the atmospheric pressure;

- L* is a shape factor and is expressed according to the following equation:

$$L^* = \frac{A_0}{P_t h} \qquad (5)$$

wherein:

- h is the blood pool's height; and
- $P_t$ is the wet perimeter.

3. The computer-implemented method of any of the preceding claims, wherein determining the drying front of the blood pool at the given time (t) further comprises determining the wet area ($A_t$) of the blood pool at the given time (t).

4. The computer-implemented method of any of the preceding claims, wherein the drying model comprises a function correlating the mass variation of the blood pool with time with the wet area ($A_t$) of the blood pool at the given time (t).

5. The computer-implemented method of claim 4, wherein the function correlating the mass variation of the blood pool with time with the wet area ($A_t$) of the blood pool at the given time (t) is expressed according to the following equation:

$$\frac{\delta m}{\delta t} = -\frac{\alpha m_0 \left[1 - \left(\frac{A_t}{A_0}\right)\right]^{\beta}}{\delta t} \qquad (6)$$

wherein:

- $\alpha$ and $\beta$ are the correlation coefficients which are constant at the environmental conditions to which the blood pool is exposed at the given time (t);
- $A_t$ is the wet area of the blood pool at the given time (t);
- $\delta t$ is the time elapsed between the given time (t) and the initial time ($t_0$);
- $\delta m$ is the mass variation of the blood pool during the time elapsed between the given time (t) and the initial time ($t_0$);
- $m_0$ is the mass of the blood pool at the initial time ($t_0$) and is expressed as according to the following equation:

$$m_0 = A_0 \rho h \qquad (7)$$

wherein:

- $\rho$ is the blood's volume weight.

6. The computer-implemented method of claim 5, wherein the time elapsed between the given time (t) and the initial time ($t_0$) is expressed according to the following equation:

EP 3 984 041 B1

$$\delta t \;=\; \frac{\alpha \, R k_B T^2 A_0^{1/2} h^{1/2} \rho \left[1 - \left(\frac{A_t}{A_0}\right)\right]^{\beta}}{\pi d^2 M P_t^{1/2} D_{blood} P_w P_a} \qquad (8)$$

7. The computer-implemented method according to any of the preceding claims, comprising the repetition of at least the steps of claim 1 a predetermined number of times.

8. System for dating a blood pool lying on a substrate, comprising:

means for determining, based on at least one picture of a blood pool lying on a substrate taken at a given time (t), the drying front of the blood pool at the given time (t); and

means for determining, based on a drying model and on the determined drying front of the blood pool at the given time (t), the time elapsed between the given time (t) and an initial time ($t_0$) at which the blood pool initiated a drying process on the substrate,

wherein the means for determining the drying front of the blood pool at the given time (t) determines the wet perimeter ($P_t$) of the blood pool at the given time (t), and

wherein the means for determining the time elapsed between the given time (t) and the initial time ($t_0$) correlates the time elapsed with a blood's diffusion coefficient ($D_{blood}$) measured for a plurality of predetermined environmental conditions and a plurality of initial blood pool conditions; and

wherein:

- the means for determining the drying front of the blood pool at the given time (t) comprises an image processing software;
- the means for determining the time elapsed between the given time (t) and the initial time ($t_0$) comprises a calculation software; and

wherein the system further includes a database comprising sets of predetermined environmental conditions comprising at least the temperature and humidity of the environment to which a blood pool is exposed, and the nature of the substrate on which a blood pool lies, and initial blood pool conditions including initial areas ($A_0$), the sets of predetermined environmental and initial blood pool conditions being associated with corresponding sets of measured blood pool parameters including blood's diffusion coefficients ($D_{blood}$), blood pool's heights (h) and correlation coefficients $\alpha$ and $\beta$,

wherein the measured blood pool parameters of each set of measured blood pool parameters are measured at the environmental and initial blood pool conditions of the associated set of predetermined environmental and initial blood pool conditions, and

wherein the correlation coefficients $\alpha$ and $\beta$ are measured for a predetermined number of blood pools having different masses, and provided by a fitting curve deduced from the superimposition of a predetermined number of corresponding variations of a normalized mass $m_t/m_0$ as functions of a normalized wet area $A_t/A_0$, wherein $m_t$ is the mass variation of the blood pool at the given time t, and $m_0$ is the mass of the blood pool at the initial time $t_0$,

**Patentansprüche**

1. Computerimplementiertes Verfahren zur Datierung einer auf einem Substrat liegenden Blutlache, die folgenden Schritte aufweisend:

- Bestimmen der Trocknungsfront der Blutlache zu einer gegebenen Zeit (t) basierend auf mindestens einem Bild einer auf einem Substrat liegenden Blutlache, das zu einer gegebenen Zeit (t) aufgenommen wurde,
- Bestimmen, basierend auf der bestimmten Trocknungsfront der Blutlache zur gegebenen Zeit (t) und auf einem Trocknungsmodell, der verstrichenen Zeit zwischen der gegebenen Zeit (t) und einer Anfangszeit ($t_0$), zu der die Blutlache einen Trocknungsprozess auf dem Substrat initiierte,

wobei das Bestimmen der Trocknungsfront der Blutlache zu der gegebenen Zeit (t) das Bestimmen des feuchten Umrisses (Pt) der Blutlache zu der gegebenen Zeit (t) aufweist, und wobei das Trocknungsmodell die verstrichene Zeit mit einem Blutdiffusionskoeffizienten ($D_{blood}$) korreliert, der für eine Vielzahl von vorgegebenen Umgebungsbedingungen und eine Vielzahl von anfänglichen Blutlachenbedingungen gemessen wurde; und wobei die verstrichene Zeit zwischen der gegebenen Zeit (t) und der Anfangszeit ($t_0$) bestimmt wird durch:

20

- Bereitstellen einer Datenbank, die Sets von vorgegebenen Umgebungsbedingungen aufweist, die mindestens die Temperatur und die Feuchtigkeit der Umgebung, der eine Blutlache ausgesetzt ist, und die Beschaffenheit des Substrats, auf dem eine Blutlache liegt, aufweisen, und anfängliche Blutlachenbedingungen, welche anfängliche Flächen ($A_0$) aufweisen, wobei die Sets von vorgegebenen Umgebungs- und anfänglichen Blutlachenbedingungen mit entsprechenden Sets von gemessenen Blutlachenparametern verbunden sind, die Blutdiffusionskoeffizienten ($D_{blood}$), Blutlachenhöhen (h) und Korrelationskoeffizienten $\alpha$ und $\beta$ aufweisen,

wobei die gemessenen Parameter der Blutlache jedes Sets von gemessenen Parametern der Blutlache bei den Umgebungs- und anfänglichen Blutlachenbedingungen des zugehörigen Sets von vorgegebenen Umgebungs- und anfänglichen Blutlachenbedingungen gemessen werden, und
wobei die Korrelationskoeffizienten $\alpha$ und $\beta$ für eine vorgegebene Anzahl von Blutlachen mit unterschiedlichen Massen gemessen werden und von einer Anpassungskurve gegeben werden, die aus der Überlagerung einer vorbestimmten Anzahl entsprechender Variationen einer normalisierten Masse ($m_t/m_0$) als Funktionen einer normalisierten Feuchtfläche ($A_t/A_0$) abgeleitet wird, wobei $m_t$ die Massenvariation der Blutlache zur gegebenen Zeit t ist und $m_0$ die Masse der Blutlache zur Anfangszeit $t_0$ ist,

- Bestimmen der Umgebungsbedingungen, die die Temperatur und die Feuchtigkeit der Umgebung, der die Blutlache zur gegebenen Zeit (t) ausgesetzt ist, und die Art des Substrats, auf dem die Blutlache liegt, aufweisen, und einer anfänglichen Fläche ($A_0$) der Blutlache, die der Fläche der Blutlache zur Anfangszeit ($t_0$) entspricht,
- Vergleichen der bestimmten Umgebungsbedingungen und der bestimmten anfänglichen Fläche ($A_0$) mit den Sets der vorgegebenen Umgebungs- und anfänglichen Blutlachenbedingungen, und
- Bestimmen eines Sets von gemessenen Blutlachenparametern, das den Blutdiffusionskoeffizienten ($D_{blood}$), die Höhe (h) der Blutlache und die Korrelationskoeffizienten $\alpha$ und $\beta$ aufweist, die dem Set von vorgegebenen Umgebungs- und anfänglichen Blutlachenbedingungen zugeordnet sind, die mit den bestimmten Umgebungsbedingungen und der bestimmten Anfangsfläche ($A_0$) identisch sind.

2. Das computerimplementierte Verfahren nach Anspruch 1, wobei das Trocknungsmodell eine Funktion aufweist, die eine Korrelation zwischen der zur gegebenen Zeit (t) und der Anfangszeit ($t_0$) verstrichenen Zeit und dem Blutdiffusionskoeffizienten ($D_{blood}$) herstellt, wobei der Blutdiffusionskoeffizient während der zwischen der zur gegebenen Zeit (t) und der Anfangszeit ($t_0$) verstrichenen Zeit für dieselben Umgebungsbedingungen einen im Wesentlichen konstanten Wert aufweist,

wobei der Blutdiffusionskoeffizient gemäß der folgenden Gleichung ausgedrückt wird:

$$D_{blood} = K_i L_k L^{*0.5} \qquad (1)$$

wobei:

- $D_{blood}$ der Diffusionskoeffizient des Blutes ist;
- $K_i$ ein Koeffizient für den Übergang vom flüssigen in den gasförmigen Zustand ist und durch die folgende Gleichung ausgedrückt wird:

$$K_i = J^* \frac{RT}{MP_w} \qquad (2)$$

wobei:

- M das Molekulargewicht von Wasser ist;
- $P_w$ der Sättigungsdampfdruck des Wassers an der Oberfläche der Blutlache ist;
- R die ideale Gaskonstante ist;
- T die Temperatur der Umgebung ist, der die Blutlache ausgesetzt ist; und
- J* die Verdunstungsrate der Blutlache ist und gemäß der folgenden Gleichung ausgedrückt wird:

$$J^* = \frac{\delta m}{\delta t} \times \frac{1}{A_0} \qquad (3)$$

wobei:

- $\delta m/\delta t$ die Massenänderung der Blutlache im Lauf der Zeit ist; und
- $A_0$ die anfängliche Fläche der Blutlache zur Anfangszeit ($t_0$) ist;

- $L_k$ die Knudsen-Schicht ist und durch die folgende Gleichung ausgedrückt wird:

$$L_k = \frac{k_B T}{\pi d^2 P_a} \qquad (4)$$

wobei:

- $k_B$ die Boltzmann-Konstante ist;
- $d$ der Moleküldurchmesser von Wassermolekülen ist; und
- $P_a$ der atmosphärische Druck ist;

- $L^*$ ist ein Formfaktor und wird gemäß der folgenden Gleichung ausgedrückt:

$$L^* = \frac{A_0}{P_t h} \qquad (5)$$

wobei:

- $h$ die Höhe der Blutlache ist; und
- $P_t$ der feuchte Umriss ist.

3. Das computerimplementierte Verfahren nach einem der vorhergehenden Ansprüche, wobei das Bestimmen der Trocknungsfront der Blutlache zur gegebenen Zeit (t) ferner das Bestimmen der feuchten Fläche (At) der Blutlache zur gegebenen Zeit (t) aufweist.

4. Das computerimplementierte Verfahren nach einem der vorhergehenden Ansprüche, wobei das Trocknungsmodell eine Funktion aufweist, die die Massenänderung der Blutlache mit der Zeit mit der feuchten Fläche (At) der Blutlache zur gegebenen Zeit (t) korreliert.

5. Das computerimplementierte Verfahren nach Anspruch 4, wobei die Funktion, die die Massenänderung der Blutlache mit der Zeit mit der feuchten Fläche der Blutlache zur gegebenen Zeit (t) korreliert, gemäß der folgenden Gleichung ausgedrückt wird:

$$\frac{\delta m}{\delta t} = -\frac{\alpha\, m_0 \left[1 - \left(\frac{A_t}{A_0}\right)\right]^{\beta}}{\delta t} \qquad (6)$$

wobei:

- $\alpha$ und $\beta$ Korrelationskoeffizienten sind, die unter den Umgebungsbedingungen, denen die Blutlache zur gegebenen Zeit (t) ausgesetzt ist, konstant sind;
- $A_t$ die feuchte Fläche der Blutlache zur gegebenen Zeit (t) ist;
- $\delta t$ die Zeit ist, die zwischen der gegebenen Zeit (t) und der Anfangszeit ($t_0$) verstrichen ist;
- $\delta m$ die Massenänderung der Blutlache während der verstrichenen Zeit zwischen der gegebenen Zeit (t) und der Anfangszeit ($t_0$) ist;
- $m_0$ die Masse der Blutlache zur Anfangszeit ($t_0$) ist und gemäß der folgenden Gleichung ausgedrückt wird:

$$m_0 = A_0 \rho h \qquad (7)$$

wobei:

- $\rho$ das Volumengewicht des Blutes ist.

**6.** Das computerimplementierte Verfahren nach Anspruch 5, wobei die verstrichene Zeit zwischen der gegebenen Zeit (t) und der Anfangszeit ($t_0$) durch die folgende Gleichung ausgedrückt wird:

$$\delta t = \frac{\alpha \, R k_B T^2 A_0^{1/2} h^{1/2} \rho [1 - \left(\frac{A_t}{A_0}\right)]^{\beta}}{\pi d^2 M P_t^{1/2} D_{blood} P_w P_a} \qquad (8)$$

**7.** Das computerimplementierte Verfahren nach einem der vorhergehenden Ansprüche, aufweisend die Wiederholung mindestens der Schritte nach Anspruch 1 in einer vorgegebenen Anzahl von Wiederholungen.

**8.** System zur Datierung einer auf einem Substrat liegenden Blutlache, aufweisend:

Mittel zum Bestimmen der Trocknungsfront der Blutlache zur gegebenen Zeit (t) basierend auf mindestens einem zu einer gegebenen Zeit (t) aufgenommenen Bild einer auf einem Substrat liegenden Blutlache; und
Mittel zum Bestimmen der verstrichenen Zeit zwischen der gegebenen Zeit (t) und einer Anfangszeit (t0), zu der die Blutlache einen Trocknungsprozess auf dem Substrat initiierte, basierend auf einem Trocknungsmodell und auf der bestimmten Trocknungsfront der Blutlache zu der gegebenen Zeit (t),
wobei das Mittel zum Bestimmen der Trocknungsfront der Blutlache zur gegebenen Zeit (t) derart konfiguriert ist, dass der feuchte Umriss ($P_t$) der Blutlache zur gegebenen Zeit (t) bestimmt wird; und
wobei das Mittel zum Bestimmen der verstrichenen Zeit zwischen der gegebenen Zeit (t) und der Anfangszeit ($t_0$) die verstrichene Zeit mit einem Blutdiffusionskoeffizienten ($D_{blood}$) korreliert, der für eine Vielzahl von vorgegebenen Umgebungsbedingungen und eine Vielzahl von anfänglichen Blutlachenbedingungen gemessen wurde; und

wobei:

- das Mittel zum Bestimmen der Trocknungsfront der Blutlache zur gegebenen Zeit (t) eine Bildverarbeitungssoftware aufweist;
- das Mittel zum Bestimmen der zwischen der gegebenen Zeit (t) und der Anfangszeit ($t_0$) verstrichenen Zeit eine Berechnungssoftware aufweist; und

wobei das System ferner eine Datenbank aufweist, die Sets von vorgegebenen Umgebungsbedingungen aufweist, die zumindest die Temperatur und Feuchtigkeit der Umgebung, der eine Blutlache ausgesetzt ist, und die Beschaffenheit des Substrats, auf dem eine Blutlache liegt, sowie anfängliche Blutlachenbedingungen einschließlich anfänglicher Flächen ($A_0$) aufweisen, wobei die Sets von vorgegebenen Umgebungs- und anfänglichen Blutlachenbedingungen mit entsprechenden Sets von gemessenen Blutlachenparametern einschließlich Blutdiffusionskoeffizienten ($D_{blood}$), Blutlachenhöhen (h) und Korrelationskoeffizienten $\alpha$ und $\beta$ verbunden sind,
wobei die gemessenen Parameter der Blutlache jedes Sets von gemessenen Parametern der Blutlache bei den Umgebungs- und anfänglichen Blutlachenbedingungen des zugehörigen Sets von vorgegebenen Umgebungs- und anfänglichen Blutlachenbedingungen gemessen werden, und
wobei die Korrelationskoeffizienten $\alpha$ und $\beta$ für eine vorgegebene Anzahl von Blutlachen mit unterschiedlichen Massen gemessen werden und von einer Anpassungskurve gegeben werden, die aus der Überlagerung einer vorbestimmten Anzahl entsprechender Variationen einer normalisierten Masse ($m_t/m_0$) als Funktionen einer normalisierten Feuchtfläche ($A_t/A_0$) abgeleitet wird, wobei $m_t$ die Massenvariation der Blutlache zur gegebenen Zeit t ist und $m_0$ die Masse der Blutlache zur Anfangszeit $t_0$ ist.

**Revendications**

1. Procédé mis en oeuvre par ordinateur, de datation d'une flaque de sang reposant sur un substrat, comprenant :

   - la détermination, sur la base d'au moins une photo d'une flaque de sang reposant sur un substrat, prise à un instant donné (t), du front de séchage de la flaque de sang à l'instant donné (t),
   - la détermination, sur la base du front de séchage déterminé de la flaque de sang à l'instant donné (t) et d'un modèle de séchage, du temps écoulé entre l'instant donné (t) et un instant initial (t$_0$) auquel la flaque de sang a entamé un processus de séchage sur le substrat,
   dans lequel la détermination du front de séchage de la flaque de sang à l'instant donné (t) comprend la détermination du périmètre humide (Pt) de la flaque de sang à l'instant donné (t), et
   dans lequel le modèle de séchage établit une corrélation entre le temps écoulé et un coefficient de diffusion du sang (D$_{blood}$) mesuré pour une pluralité de conditions environnementales prédéterminées et une pluralité de conditions initiales de flaque de sang ; et
   dans lequel le temps écoulé entre l'instant donné (t) et l'instant initial (t$_0$) est déterminé par :

   - la fourniture d'une base de données comprenant des ensembles de conditions environnementales prédéterminées comprenant au moins la température et l'humidité de l'environnement auquel une flaque de sang est exposée, et la nature du substrat sur lequel repose une flaque de sang, et des conditions initiales de flaque de sang comprenant des aires initiales (A$_0$), les ensembles de conditions environnementales prédéterminées et de conditions initiales de flaque de sang étant associés à des ensembles correspondants de paramètres de flaque de sang mesurés comprenant des coefficients de diffusion du sang (D$_{blood}$), hauteurs de flaque de sang (h), et coefficients de corrélation $\alpha$ et $\beta$,

     dans lequel les paramètres de flaque de sang mesurés de chaque ensemble de paramètres de flaque de sang mesurés sont mesurés dans les conditions environnementales et initiales de flaque de sang de l'ensemble associé de conditions environnementales prédéterminées et de conditions initiales de flaque de sang , et
     dans lequel les coefficients de corrélation $\alpha$ et $\beta$ sont mesurés pour un nombre prédéterminé de flaques de sang ayant des masses différentes, et fournis par une courbe d'ajustement déduite de la superposition d'un nombre prédéterminé de variations correspondantes d'une masse normalisée (m$_t$/m$_0$) en fonction d'une aire humide normalisée (A$_t$/A$_0$), où m$_t$ est la variation de masse de la flaque de sang à l'instant donné t, et m$_0$ est la masse de la flaque de sang à l'instant initial (t$_0$),

   - la détermination des conditions environnementales comprenant la température et l'humidité de l'environnement auquel la flaque de sang est exposée à l'instant donné (t) et la nature du substrat sur lequel repose la flaque de sang, et d'une aire initiale (A$_0$) de la flaque de sang correspondant à l'aire de la flaque de sang à l'instant initial (t$_0$),
   - la comparaison des conditions environnementales déterminées et de l'aire initiale déterminée (A$_0$) avec les ensembles de conditions environnementales prédéterminées et de conditions initiales de flaque de sang, et
   - la détermination d'un ensemble de paramètres de flaque de sang mesurés comprenant le coefficient de diffusion du sang (D$_{blood}$), la hauteur de la flaque de sang (h) et les coefficients de corrélation $\alpha$ et $\beta$, qui sont associés à l'ensemble des conditions environnementales prédéterminées et de conditions initiales de flaque de sang identiques aux conditions environnementales déterminées et à l'aire initiale déterminée (A$_0$).

2. Procédé mis en oeuvre par ordinateur selon la revendication 1, dans lequel le modèle de séchage comprend une fonction corrélant le temps écoulé entre l'instant donné (t) et l'instant initial (t$_0$) avec le coefficient de diffusion du sang (D$_{blood}$), le coefficient de diffusion du sang ayant une valeur sensiblement constante pendant le temps écoulé entre l'instant donné (t) et l'instant initial (t$_0$) pour les mêmes conditions environnementales,

   dans lequel le coefficient de diffusion du sang est exprimé selon l'équation suivante :

$$D_{blood} = K_i L_k L^{*0.5} \qquad (1)$$

   dans laquelle :

- $D_{blood}$ est le coefficient de diffusion du sang ;
- $K_i$ est un coefficient de transfert de l'état liquide à l'état gazeux et s'exprime selon l'équation suivante :

$$K_i = J^* \frac{RT}{MP_w} \qquad (2)$$

dans laquelle :

- M est le poids moléculaire de l'eau ;
- $P_w$ est la pression de vapeur saturante de l'eau à la surface de la flaque de sang ;
- R est la constante des gaz parfaits ;
- T est la température de l'environnement auquel la flaque de sang est exposée ; et
- J* est le taux d'évaporation de la flaque de sang et s'exprime selon l'équation suivante :

$$J^* = \frac{\delta m}{\delta t} \times \frac{1}{A_0} \qquad (3)$$

dans laquelle :

- $\delta m/\delta t$ est la variation de la masse de la flaque de sang en fonction du temps ; et
- $A_0$ est l'aire de la flaque de sang à l'instant initial ($t_0$) ;

- $L_k$ est la couche de Knudsen et s'exprime selon l'équation suivante :

$$L_k = \frac{k_B T}{\pi d^2 P_a} \qquad (4)$$

dans laquelle :

- $k_B$ est la constante de Boltzmann ;
- d est le diamètre moléculaire des molécules d'eau ; et
- $P_a$ est la pression atmosphérique ;

- L* est un facteur de forme et s'exprime selon l'équation suivante :

$$L^* = \frac{A_0}{P_t h} \qquad (5)$$

dans laquelle :

- h est la hauteur de la flaque de sang ; et
- $P_t$ est le périmètre humide.

3. Procédé mis en oeuvre par ordinateur selon l'une quelconque des revendications précédentes, dans lequel la détermination du front de séchage de la flaque de sang à l'instant donné (t) comprend en outre la détermination de l'aire humide (At) de la flaque de sang à l'instant donné (t).

4. Procédé mis en oeuvre par ordinateur selon l'une quelconque des revendications précédentes, dans lequel le modèle de séchage comprend une fonction corrélant la variation de masse de la flaque de sang dans le temps avec l'aire humide (At) de la flaque de sang à l'instant donné (t).

5. Procédé mis en oeuvre par ordinateur selon la revendication 4, dans lequel la fonction corrélant la variation de masse de la flaque de sang dans le temps avec l'aire humide (At) de la flaque de sang à l'instant donné (t) est exprimée selon l'équation suivante :

$$\frac{\delta m}{\delta t} = -\frac{\alpha\, m_0 \left[1 - \left(\frac{A_t}{A_0}\right)\right]^{\beta}}{\delta t} \qquad (6)$$

dans laquelle :

- $\alpha$ et $\beta$ sont les coefficients de corrélation qui sont constants dans les conditions environnementales auxquelles la flaque de sang est exposée à l'instant donné (t) ;
- $A_t$ est l'aire humide de la flaque de sang à l'instant donné (t) ;
- $\delta t$ est le temps écoulé entre l'instant donné (t) et l'instant initial ($t_0$) ;
- $\delta m$ est la variation de masse de la flaque de sang pendant le temps écoulé entre l'instant donné (t) et l'instant initial ($t_0$) ;
- $m_0$ est la masse de la flaque de sang à l'instant initial ($t_0$), exprimée selon l'équation suivante :

$$m_0 = A_0 \rho h \qquad (7)$$

dans laquelle :

- $\rho$ est la masse volumique du sang.

6. Procédé mis en oeuvre par ordinateur selon la revendication 5 dans lequel le temps écoulé entre l'instant donné (t) et l'instant initial ($t_0$) est exprimé selon l'équation suivante :

$$\delta t = \frac{\alpha\, R k_B T^2 A_0^{1/2} h^{1/2} \rho [1 - \left(\frac{A_t}{A_0}\right)]^{\beta}}{\pi d^2 M P_t^{1/2} D_{blood} P_w P_a} \qquad (8)$$

7. Procédé mis en oeuvre par ordinateur selon l'une quelconque des revendications précédentes, comprenant la répétition d'au moins les étapes de la revendication 1, un nombre prédéterminé de fois.

8. Système de datation d'une flaque de sang reposant sur un substrat, comprenant :

des moyens pour déterminer, sur la base d'au moins une photo d'une flaque de sang reposant sur un substrat, prise à un instant donné (t), le front de séchage de la flaque de sang à l'instant donné (t) ; et
des moyens pour déterminer, sur la base d'un modèle de séchage et du front de séchage déterminé de la flaque de sang à l'instant donné (t), le temps écoulé entre l'instant donné (t) et un instant initial ($t_0$) auquel la flaque de sang a entamé un processus de séchage sur le substrat,
dans lequel le moyen pour déterminer le front de séchage de la flaque de sang à l'instant donné (t) détermine le périmètre humide ($P_t$) de la flaque de sang à l'instant donné (t), et
dans lequel le moyen pour déterminer le temps écoulé entre l'instant donné (t) et l'instant initial ($t_0$) met en corrélation le temps écoulé avec un coefficient de diffusion du sang ($D_{blood}$) mesuré pour une pluralité de conditions environnementales prédéterminées et une pluralité de conditions initiales de flaque de sang ; et
dans lequel :

- le moyen pour déterminer le front de séchage de la flaque de sang à l'instant donné (t) comprend un logiciel de traitement d'images ;
- le moyen pour déterminer le temps écoulé entre l'instant donné (t) et l'instant initial ($t_0$) comprend un logiciel de calcul ; et

dans lequel le système comprend en outre une base de données comprenant des ensembles de conditions environnementales prédéterminées comprenant au moins la température et l'humidité de l'environnement auquel une flaque de sang est exposée, et la nature du substrat sur lequel repose une flaque de sang, ainsi que des conditions initiales de flaque de sang comprenant des aires initiales ($A_0$), les ensembles de conditions environnementales prédéterminées et de conditions initiales de flaque de sang étant associés à des ensembles correspondants de paramètres de flaque de sang mesurés comprenant des coefficients de diffusion du sang

($D_{blood}$), hauteurs de flaque de sang (h) et coefficients de corrélation $\alpha$ et $\beta$, dans lequel les paramètres de flaque de sang mesurés de chaque ensemble de paramètres de flaque de sang mesurés sont mesurés dans les conditions environnementales et initiales de flaque de sang de l'ensemble associé de conditions environnementales prédéterminées et de conditions initiales de flaque de sang, et

dans lequel les coefficients de corrélation $\alpha$ et $\beta$ sont mesurés pour un nombre prédéterminé de flaques de sang ayant des masses différentes, et fournis par une courbe d'ajustement déduite de la superposition d'un nombre prédéterminé de variations correspondantes d'une masse normalisée ($m_t/m_0$) en fonction d'une aire humide normalisée ($A_t/A_0$), où (mt) est la variation de masse de la flaque de sang à l'instant donné (t), et ($m_0$) est la masse de la flaque de sang à l'instant initial ($t_0$).

FIG.1A

FIG.1B

FIG.1C

FIG.2

FIG.3

FIG.4

FIG.5

FIG.6

Legend:
- H=3.0%, T=21°C, Unvarnished Wood
- H=3.0%, T=21°C, Varnished Wood
- H=4.0%, T=21°C, Unvarnished Wood
- H=4.0%, T=21°C, Varnished Wood
- H=5.0%, T=21°C, Unvarnished Wood

X-axis: Percentage of water left in the blood pools

Y-axis: $(K_i L_k L^{*0/5})(m^2, s^{-1})$

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LAAN et al.** Morphology of drying blood pools. *Forensic Sci. Int.,* 2016, vol. 267, 104-109
- **CHOI et al.** Highly sensitive and accurate estimation of bloodstain age using smartphone. *Biosens. Bioelectron.,* 2019, vol. 130, 414-419

- **THANAKIATKRAI et al.** Age estimation of bloodstains using smartphones and digital image analysis. *Forensic Sci. Int.,* 2013, vol. 233, 288-297